# EUROPEAN PATENT APPLICATION

(11) **EP 4 597 378 A1**
(43) Date of publication of application: **06.08.2025**
(21) Application number: 22961053.0
(22) Date of filing: 22.12.2022
(51) Int. Cl.: G06Q 10/04, G06Q 50/12

(54) **PREDICTION DEVICE, LEARNING DEVICE, AND LEARNING METHOD**

(30) Priority: 26.09.2022 JP 2022152725
(71) Applicant: Kikkoman Corporation, Noda-shi, Chiba 278-8601 (JP)
(72) Inventor: TATEMICHI, Yuki, Noda-shi, Chiba 278-8601 (JP); TOHARA, Miho, Noda-shi, Chiba 278-8601 (JP)
(74) Representative: Epping - Hermann - Fischer
(86) International application number: PCT/JP2022/047266
(87) International publication number: WO 2024/069994

(57) **Abstract**

Provided is a prediction apparatus including: an input section; a processing section; and an output section. The processing section extracts an index score which is inputted into a compatibility prediction model for an ingredient and a seasoning. The output section outputs a compatibility score as a predicted value of compatibility between the ingredient and the seasoning, where the compatibility score has been outputted from the compatibility prediction model by inputting the index score, which has been extracted by the processing section, into the compatibility prediction model.

## Description

### Technical Field

The present disclosure relates to a prediction apparatus, a learning apparatus, and a learning method.

### Background Art

Seasonings used for cooking are preferably selected in accordance with ingredients having a good compatibility with the seasonings in consideration of the effect on the finished dishes.

Compatibility is a concept extremely important to a person skilled in the art in terms of considering a combination of an ingredient and a seasoning. In general, it is said that in a case where there is a good compatibility, a pleasant flavor is newly generated or enhanced, or an unpleasant flavor is suppressed. It is said, on the other hand, that in a case where there is a bad compatibility, a pleasant flavor is suppressed or an unpleasant flavor is newly generated or enhanced (see, for example, Non-Patent Literature (hereinafter referred to as "NPL") 1).

Note that, as a method that allows compatibility between *sake* (Japanese rice wine) and a dish to be determined even without specialized knowledge or experience, a method, although not a seasoning-related technique, in which an expert evaluates the characteristics of the *sake* with four levels of tastes and aromas in advance and the determination results are displayed on a product label has been proposed (see, for example, Patent Literature (hereinafter referred to as "PTL") 1).

### Citation List

### Patent Literature

PTL 1
Japanese Patent No. 3559339

### Non-Patent Literature

NPL 1
Tamura TAKAYUKI, "Ryouri to wain no mariage no kagaku (Science of Mariage between Dish and Wine)", Kagaku to Seibutsu, 2010, Vol. 48, No. 10, p. 668-670, Publication Date: October 3, 2011, Online ISSN 1883-6852, Print ISSN 0453-073X, https://doi.org/10.1271/kagakutoseibutsu.48.668, https://www.jstage.jst.go.jp/article/kagakutoseibutsu/48/10/48_10_668/_article/-char/ja

### Summary of Invention

### Technical Problem

Incidentally, persons who have a meal have various preferences, such as meat-eaters and vegetarians. For example, there are countries, such as India, where there is no culture of eating beef and the main ingredients are vegetables, beans, fish, or the like. Accordingly, in a case where compatibility is predicted only based on ingredients, seasonings, and common characteristics without considering the preferences of persons who have a meal, accurate compatibility prediction may not be performed.

An object of the present disclosure is to provide a prediction apparatus, a learning apparatus, and a learning method each capable of predicting compatibility between an ingredient and a seasoning in consideration of the preferences of persons who have a meal.

### Solution to Problem

A prediction apparatus of the present disclosure includes: an input section; a processing section; and an output section. The input section allows information on an ingredient and a seasoning to be inputted into the input section. The processing section extracts an index score, which is based on a first index calculated based on data on a flavor in the ingredient and a second index calculated based on data on a flavor in the seasoning and is inputted into a compatibility prediction model for the ingredient and the seasoning, based on the information inputted into the input section, where the compatibility prediction model has been learned based on the index score and a compatibility score that indicates compatibility between the ingredient and the seasoning based on a pre-aggregated evaluation result of the compatibility between the ingredient and the seasoning. The output section outputs the compatibility score as a predicted value of the compatibility between the ingredient and the seasoning, where the compatibility score has been outputted from the compatibility prediction model by inputting the index score, which has been extracted by the processing section, into the compatibility prediction model.

A learning apparatus of the present disclosure includes: a calculation section; and a generation section. The calculation section calculates an index score based on a first index calculated based on data on a flavor in an ingredient and a second index calculated based on data on a flavor in a seasoning. The generation section performs learning based on the index score and a compatibility score, which indicates compatibility between the ingredient and the seasoning based on a pre-aggregated evaluation result of the compatibility between the ingredient and the seasoning, and generates a compatibility prediction model for the ingredient and the seasoning.

A learning method of the present disclosure includes: calculating an index score based on a first index calculated based on data on a flavor in an ingredient and a second index calculated based on data on a flavor in a seasoning; and performing learning based on the index score and a compatibility score, which indicates compatibility between the ingredient and the seasoning based on a pre-aggregated evaluation result of the compatibility between the ingredient and the seasoning, and generating a compatibility prediction model for the ingredient and the seasoning.

### Advantageous Effects of Invention

According to the present disclosure, it is possible to predict compatibility between an ingredient and a seasoning in consideration of the preferences of persons who have a meal.

### Brief Description of Drawings

FIG. 1 is a block diagram illustrating a learning apparatus according to Embodiment 1 of the present disclosure;
FIG. 2 illustrates exemplary calculation results of OAV conversion scores in ingredients and seasonings, respectively;
FIG. 3 is a block diagram illustrating a prediction apparatus according to Embodiment 1 of the present disclosure;
FIG. 4 is a flowchart illustrating an operation example of learning control in the learning apparatus;
FIG. 5 is a block diagram illustrating a learning apparatus according to Embodiment 2 of the present disclosure;
FIG. 6 is a diagram provided for describing a tree diagram in a hierarchical cluster analysis;
FIG. 7 illustrates a list of grouped ingredients and seasonings;
FIG. 8 is a table illustrating comparison results of experiments of compatibility prediction accuracy between Embodiments 1 and 2;
FIG. 9 is a table illustrating results of experiments of compatibility prediction accuracy in Embodiment 3;
FIG. 10 illustrates results of experiments of compatibility prediction accuracy according to a variation;
FIG. 11A illustrates results of a questionnaire survey of (1);
FIG. 11B illustrates results of a questionnaire survey of (2);
FIG. 11C illustrates results of a questionnaire survey of (3);
FIG. 12A illustrates a breakdown of the respective numbers of Indian chefs who select one of pieces of fried rice in (2), where the Indian chefs have selected third fried rice in (1);
FIG. 12B illustrates a breakdown of the respective numbers of Indian chefs who select one of the pieces of fried rice in (2), where the Indian chefs have selected first fried rice in (1); and
FIG. 13 illustrates aggregate results of scores of pieces of fried rice selected in each group.

### Description of Embodiment

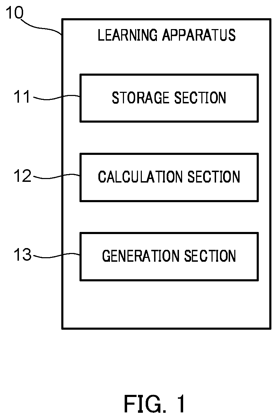

Hereinafter, Embodiment 1 of the present disclosure will be described with reference to the accompanying drawings. FIG. 1 is a block diagram illustrating a learning apparatus according to Embodiment 1 of the present disclosure.

As illustrated in FIG. 1, learning apparatus 10 is an apparatus (for example, a terminal apparatus such as a personal computer) that performs learning based on an index score related to flavors in an ingredient and a seasoning and a compatibility score based on a pre-aggregated evaluation of compatibility between the ingredient and the seasoning and generates a compatibility prediction model for the ingredient and the seasoning.

Learning apparatus 10 includes a central processing unit (CPU), a read only memory (ROM), a random access memory (RAM), and input/output circuitry that are not illustrated, and generates the compatibility prediction model described above based on a program set in advance. Learning apparatus 10 includes storage section 11, calculation section 12, and generation section 13.

Storage section 11 stores a compatibility prediction model to be learned, and the index score described above and the compatibility score described above as teacher data.

The compatibility score is a score of compatibility between one of a plurality of ingredients and one of a plurality of seasonings based on a pre-aggregated evaluation of compatibility between the ingredient and the seasoning.

The plurality of ingredients may be arbitrarily settable. For example, 12 kinds of ingredients: cottage cheese, cauliflower, mushroom, green pepper, tomato, coconut, chicken, spinach, aromatic rice, onion, mozzarella cheese, and mango are set as the ingredients in the present embodiment.

The plurality of seasonings may be arbitrarily settable. For example, 17 kinds of seasonings: tomato paste, black pepper, butter, garlic, cumin, ghee, turmeric, ginger, soy sauce, dill, bay leaf, milk, clove, nutmeg, white wine, lemon juice, and buttermilk are set as the seasonings in the present embodiment.

The pre-aggregated evaluation of compatibility between an ingredient and a seasoning is an evaluation based on the viewpoints of a plurality of persons in compatibility between the ingredient and the seasoning, and may be, for example, based on a questionnaire(s) acquired from a plurality of persons in the food industry. The plurality of persons in the food industry may be, for example, persons with cooking-related knowledge, such as cooks and researchers of cooking, in a specific country (for example, India, Brazil, U.S.A., or the like) in its entirety or a specific region (for example, the Kanto region, the Kansai region, or the like in the case of Japan) in its entirety.

The pre-aggregated evaluation of compatibility between an ingredient and a seasoning may be performed, for example, by keeping scores with a plurality of levels for each combination of a plurality of ingredients and a plurality of seasonings.

For example, the scores with a plurality of levels may be such scores that become higher as the evaluation becomes better, or may be such scores that become higher as the evaluation becomes worse. In the present embodiment, the scores for a plurality of levels are exemplified with nine levels of scores that become higher as the evaluation becomes better: 1: Worst pair, 2: Terrible pair, 3: Bad pair, 4: Weak pair, 5: Can't say, 6: Fair pair, 7: Good pair, 8: Great pair, and 9: Perfect pair.

The average value of each score for each combination of ingredients and seasonings by all persons who perform the pre-aggregated evaluation of compatibility between an ingredient and a seasoning as described above may be the compatibility score in a combination of an ingredient and a seasoning.

Storage section 11 stores each compatibility score of every combination of a plurality of ingredients and a plurality of seasonings. In the present embodiment, storage section 11 stores compatibility scores according to a total of 204 combinations of the 12 kinds of ingredients and the 17 kinds of seasonings.

Calculation section 12 calculates each index score of a plurality of ingredients and a plurality of seasonings.

The index score is a score based on a first index and a second index that are indexes related to flavors in an ingredient or a seasoning. The first index is an index calculated based on data on a flavor in an ingredient, and the second index is an index calculated based on data on a flavor in a seasoning. The first and second indexes may be, for example, odor activity values (OAVs) known as an index indicating the importance of an aroma component.

The OAV is a value obtained by dividing the concentration (ppm) of an aroma component included in an ingredient or a seasoning by a threshold value (ppm). The concentration of an aroma component may be a value actually measured from an ingredient or a seasoning, or may be a value acquired from an article on the concentration(s) of an aroma component(s) or an organization including a database of the concentrations of aroma components. The threshold value may be set to an arbitrary value such as, for example, the concentration of an aroma component, at which a person no longer perceives the aroma component, or may be a value acquired from the article as described above or the organization including the database as described above.

For example, the concentration of an aroma component and the threshold value as described above may be acquired from a predetermined database (Pub Chem: https://pubchem.ncbi.nlm.nih.gov/, ChemSpider: http://www.chemspider.com/).

An aroma component is classified into, for example, a plurality of flavor types. Learning apparatus 10 calculates the OAVs for each ingredient and each seasoning for each of a plurality of flavor types. In other words, a plurality of first indexes is calculated in accordance with a plurality of ingredients, respectively, a plurality of second indexes is calculated in accordance with a plurality of seasonings, respectively, and a plurality of index scores is calculated in accordance with a plurality of flavor types, respectively.

The plurality of flavor types represents a plurality of aroma components into which an aroma component is classified by aroma qualities. The plurality of flavor types may be classified as, for example, 13 kinds: Fruit, Floral, Green, Herb, Sulfur, Caramel, Roast, Nut, Wood, Spice, Cheese, Animal, and Chemical.

For example, in a case where the concentration of an aroma component of a predetermined flavor type (for example, Fruit) in a predetermined ingredient is A1 and the threshold value is B1, the OAV (first index) according to the predetermined flavor type in the predetermined ingredient is A1/B1. Further, in a case where the concentration of an aroma component of a predetermined flavor type in a predetermined seasoning is A2 and the threshold value is B2, the OAV (second index) according to the predetermined flavor type in the predetermined seasoning is A2/B2.

Note that, the flavor types described above indicate examples that are classified, for example, based on descriptors acquired from a database of the Federal Emergency Management Agency of the United States of America and a database of the Good Scents Company, but the present disclosure is not limited thereto and the flavor types described above may be classified by a method other than the method described above.

Specifically, calculation section 12 calculates the OAV for each substance included in an ingredient or a seasoning for each flavor type and totals up the OAVs for the respective substances. It is assumed that the resulting total value of OAVs is the OAV for each flavor type in the ingredient or the seasoning. The substances included in an ingredient or a seasoning indicate, for example, every substance, such as acetic acid, ethyl acetate, hexanal, and HEMF, included in the ingredient or the seasoning. For example, in a case where a predetermined seasoning includes M1 to M9, the respective OAVs for M1 to M9 are calculated for each flavor type, and the total value of OAVs for each flavor type is the OAV for each flavor type in the predetermined seasoning.

Since there are 13 kinds of flavor types in the present embodiment, 13 OAVs are obtained for one ingredient or one seasoning. That is, each of a total of 29 kinds of ingredients and seasonings: the 12 kinds of ingredients and the 17 kinds of seasonings includes 13 OAVs.

Further, apart from the OAV for each flavor type, an OAV for an aroma component that is an off-flavor may be calculated.

The off-flavor is an aroma component with a relatively high proportion perceived as an unpleasant odor by persons, and can be arbitrarily set as, for example, a raw smell of meat or fish, a sour smell of vinegar, a bitter smell of green pepper, or the like.

Note that, an aroma component set as an off-flavor may or may not be a component included in the aroma components classified as the flavor types described above.

Calculation section 12 may also calculate the OAV for an off-flavor in each substance included in an ingredient or a seasoning in the same manner as described above, and the total value of the OAVs for the respective substances included in the ingredient or the seasoning may be the OAV for the off-flavor in the ingredient or the seasoning.

For example, in a case where the concentration of an aroma component in a substance according to an off-flavor in a predetermined ingredient is A3 and the threshold value is B3, the OAV for the substance according to the off-flavor in the predetermined ingredient is A3/B3. Then, the OAV is calculated for each substance, and the total value of the OAVs thereof is the OAV for the off-flavor in the predetermined ingredient.

For example, calculation section 12 converts the 13 OAVs and the OAV for the off-flavor in each of ingredients and seasonings into logarithms (for example, common logarithms), converts every value equal to or less than -1 to -1, and performs processing of adding 1. The processing of converting each total value of OAVs into a common logarithm is processing for realizing a simple number (score) such as 1, 2, 3, or the like. For example, as the concentration of an aroma component in a predetermined ingredient or seasoning becomes greater than the threshold value, the OAV become greater and the score after the conversion becomes greater. Further, in a case where the number after the conversion is equal to or less than -1, the concentration of an aroma component in a predetermined ingredient or seasoning is equal to or less than 1/10 of the threshold value. Accordingly, it is possible to cause the score to be 0, that is, to cause the score in a case where the importance of the aroma component is low to 0 by converting every number into -1 and adding 1.

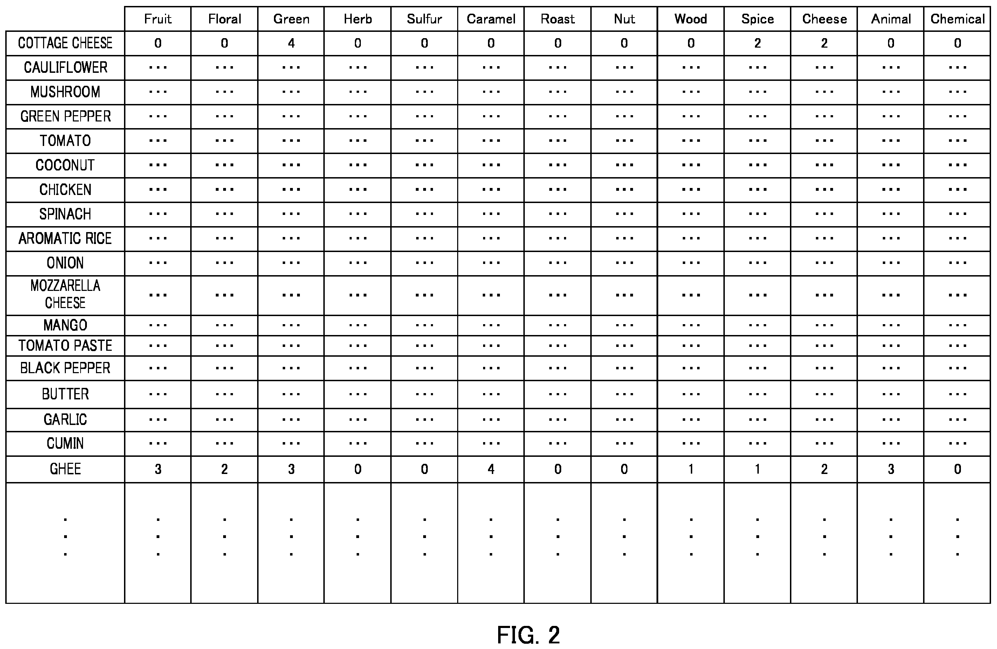

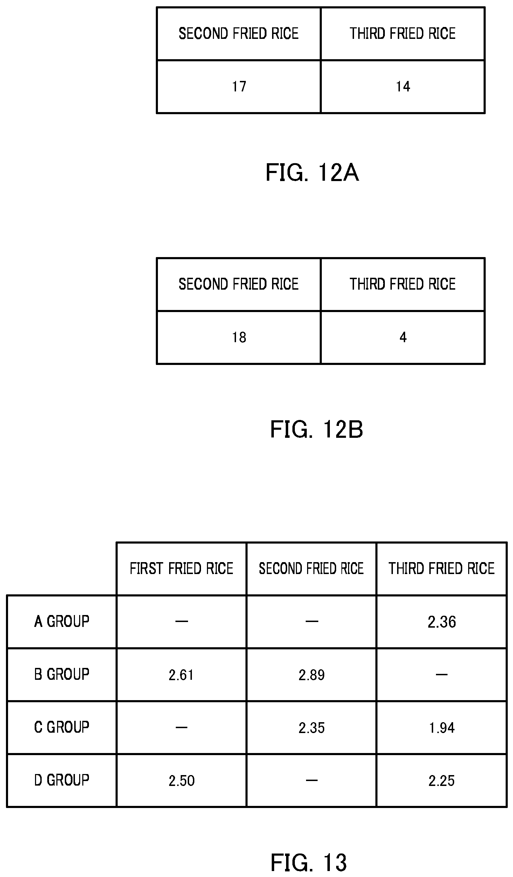

In the above-described manner, calculation section 12 calculates 13 kinds of OAV conversion scores for each of a plurality of ingredients and a plurality of seasonings. Specifically, as illustrated in FIG. 2, 13 OAV conversion scores are calculated for each of ingredients and seasonings.

Further, apart from the example illustrated in FIG. 2, calculation section 12 also calculates the OAV conversion score for an off-flavor. Accordingly, a total of 14 OAV conversion scores that are the OAV conversion score for the off-flavor and the 13 kinds of OAV conversion scores described above are calculated.

Further, calculation section 12 calculates a first score, a second score, and a third score as an index score in an arbitrary combination of the plurality of ingredients and the plurality of seasonings. That is, the index score may include the first score, the second score, and the third score.

The first score is a value obtained by adding the respective OAV conversion scores for an ingredient and a seasoning according to an arbitrary combination of the plurality of ingredients and the plurality of seasonings. For example, in a case where the OAV conversion score for the ingredient is D1 and the OAV conversion score for the seasoning is D2, the first score is D1 + D2. Specifically, in a case where the ingredient is the cottage cheese and the seasoning is the ghee in FIG. 2, the OAV conversion score for the cottage cheese is 0 and the OAV conversion score for the ghee is 3 in the flavor type of Fruit, and thus, the first score in a combination of the cottage cheese and the ghee in the flavor type of Fruit is 3. The first score is calculated for each flavor type.

The second score is the absolute value of a difference value between the respective OAV conversion scores for an ingredient and a seasoning according to an arbitrary combination of the plurality of ingredients and the plurality of seasonings. For example, in a case where the OAV conversion score for the ingredient is D1 and the OAV conversion score for the seasoning is D2, the second score is the absolute value of D1 - D2. Specifically, in a case where the ingredient is the cottage cheese and the seasoning is the ghee in FIG. 2, the OAV conversion score for the cottage cheese is 4 and the OAV conversion score for the ghee is 3 in the flavor type of Green, and thus, the second score in a combination of the cottage cheese and the ghee in the flavor type of Green is 1. The second score is calculated for each flavor type.

The third score is a value obtained by dividing the OAV conversion score for a seasoning according to an arbitrary combination of the plurality of ingredients and the plurality of seasonings by a value obtained by adding the respective off-flavor OAV conversion scores for an ingredient and the seasoning according to the arbitrary combination of the plurality of ingredients and the plurality of seasonings. For example, in a case where the OAV conversion score for the seasoning is D2, the OAV conversion score for the off-flavor in the ingredient is D3, and the OAV conversion score for the off-flavor in the seasoning is D4, the third score is D2/(D3 + D4). Specifically, in a case where the seasoning is the ghee in FIG. 2, the OAV conversion score for the ghee is 4 in the flavor type of Caramel. Further, in a case where the OAV conversion scores for the respective off-flavors when the ingredient is the cottage cheese and the seasoning is the ghee are 1 and 3, respectively, the third score in a combination of an arbitrary ingredient and the ghee in the flavor type of Caramel is 4/(1 + 3) which results in 1. The third score is calculated for each flavor type.

Calculation section 12 calculates the first score, the second score, and the third score in an arbitrary combination of the plurality of ingredients and the plurality of seasonings for each of a plurality of flavor types. That is, a total of 39 of first, second, and third scores that are 13 first scores, 13 second scores, and 13 third scores are calculated for each combination of an ingredient and a seasoning. In other words, a plurality of index scores is calculated in accordance with combinations of the first indexes for the respective ingredients and the second indexes for the respective seasonings.

In the present embodiment, there are 204 combinations of the 12 kinds of ingredients and the 17 kinds of seasonings, and thus, a total of 39 scores (index scores) are calculated for each of the 204 combinations.

Generation section 13 performs learning based on a compatibility score stored in storage section 11 and an index score calculated by calculation section 12, and generates a compatibility prediction model for an ingredient and a seasoning.

Generation section 13 generates a compatibility prediction model for an ingredient and a seasoning by using, for example, a partial least squares method. Specifically, generation section 13 generates a regression equation by using an index score as an explanatory variable and a compatibility score as an objective variable.

The index score used as the explanatory variable is the first score, the second score, and the third score according to a total of the 204 combinations of the ingredients and the seasonings, with a total of 39 × 204 = 7956 scores.

The compatibility score used as the objective variable is a total of 204 compatibility scores of the ingredients and the seasonings.

For example, generation section 13 extracts a latent variable by projecting an explanatory variable onto another space, and generates a regression equation so as to minimize an error between the extracted latent variable and an objective variable. The latent variable is extracted such that an inner product of the latent valuable and the objective variable is maximized.

For example, since there are 39 explanatory variables (index scores) for each combination of the ingredients and the seasonings and one objective variable (compatibility score) corresponding to the each combination, latent variables are extracted from the 39 explanatory variables and the corresponding objective variable in one combination, and a regression equation of y = f(x) is generated with the extracted latent variables and the objective variable, where y is the objective variable (compatibility score) and x is the latent variable (index score).

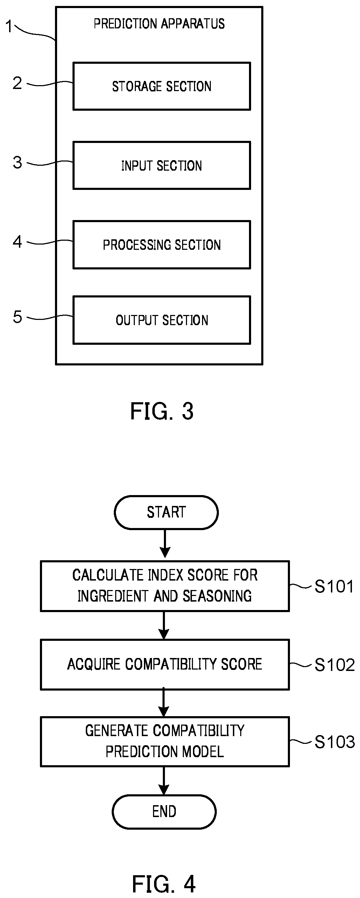

The regression equation (compatibility prediction model) generated in the above-described manner can be stored in, for example, prediction apparatus 20 and can be used. FIG. 3 is a block diagram illustrating a prediction apparatus according to Embodiment 1 of the present disclosure. Note that, the compatibility prediction model is not limited to the regression equation described above, but may be any other type of machine learning model that predicts a compatibility score from a combination of an ingredient and a seasoning.

As illustrated in FIG. 3, prediction apparatus 1 is an apparatus (for example, a terminal apparatus such as a personal computer) that outputs a predicted value of compatibility between an ingredient and a seasoning based on the compatibility prediction model having learned based on the index score and the compatibility score as described above. Prediction apparatus 1 includes storage section 2, input section 3, processing section 4, and output section 5.

Storage section 2 stores the compatibility prediction model described above. The compatibility prediction model is a learned model that is learned by learning apparatus 10.

Input section 3 is configured to allow the user to input information on an ingredient and a seasoning, compatibility between which is predicted, to input section 3.

Processing section 4 includes a CPU, a ROM, a RAM, and input/output circuitry that are not illustrated, and is configured to process inputted information on an ingredient and a seasoning to extract a predicted value of compatibility based on a program set in advance.

For example, processing section 4 applies inputted information on an ingredient and a seasoning to a compatibility prediction model, and extracts a predicted value of compatibility corresponding to a combination of the ingredient and the seasoning.

Specifically, processing section 4 refers to a table in which a combination of an ingredient and a seasoning is associated with the above-described latent variable corresponding to the combination, and extracts the latent variable corresponding to the inputted ingredient and seasoning. Then, processing section 4 substitutes the extracted latent variable in x in the regression equation of y = f(x) described above, calculates y that is the objective variable, and extracts a predicted value of compatibility.

Output section 5 outputs the predicted value of compatibility extracted by processing section 4.

Note that, the predicted value of compatibility may be calculated in advance. For example, a table in which a combination of an ingredient and a seasoning is associated with a predicted value of compatibility may be generated.

Next, an operation example of learning apparatus 10 will be described. FIG. 4 is a flowchart illustrating an operation example of learning control in learning apparatus 10.

As illustrated in FIG. 4, learning apparatus 10 calculates an index score for an ingredient and a seasoning (step S101). After step S101, learning apparatus 10 acquires a compatibility score for the ingredient and the seasoning (step S102).

After acquiring the compatibility score, learning apparatus 10 generates a compatibility prediction model based on the index score and the compatibility score (step S103). Thereafter, this control ends.

According to the present embodiment configured as described above, learning is performed based on an index score, which is based on indexes related to flavors in an ingredient and a seasoning, and a compatibility score for the ingredient and the seasoning, which is based on a pre-aggregated evaluation result of compatibility of the ingredient and the seasoning, to generate a compatibility prediction model. Then, a predicted value of the compatibility between the ingredient and the seasoning is calculated based on the compatibility prediction model.

For this reason, the present embodiment makes it possible to incorporate an element of a pre-aggregated evaluation of compatibility between an ingredient and a seasoning into prediction of compatibility between the ingredient and the seasoning, and is therefore capable of performing compatibility prediction in consideration of the preferences of persons who have a meal.

Thus, for example, by incorporating a learning result based on an evaluation result of persons in a specific region into a compatibility prediction model, the present embodiment makes it possible to perform compatibility prediction in accordance with the preferences of the persons in the region.

Further, since an index score is calculated for each of a plurality of flavor types, it is possible to perform more detailed compatibility prediction.

Further, there is a case where the first score (the value obtained by adding OAVs) is most relevant to compatibility depending on a combination of an ingredient and a seasoning. Further, there is also a case where the second score (the absolute value of the difference between OAVs) is most relevant to compatibility depending on a combination of an ingredient and a seasoning. Further, there is also a case where the third score (the value obtained by excluding the off-flavor) is most relevant to compatibility depending on a combination of an ingredient and a seasoning.

In the present embodiment, as the index score, the first score, the second score, and the third score are calculated, respectively, and thus, it is possible to collect every data related to compatibility between an ingredient and a seasoning. As a result, a more accurate predicted value of compatibility can be easily obtained.

Further, the partial least squares method is a technique characterized by sequentially using variables in descending order of importance, such as highly relevance to compatibility. For this reason, the present embodiment makes it possible to easily extract data highly relevant to compatibility due to the generation of a compatibility prediction model by the partial least squares method. For example, since the first score, the second score, and the third score are used as the explanatory variables, a score that is highly relevant to compatibility is extracted as the latent variable from the first score, the second score, and the third score. As a result, the regression equation y = f(x), where x is a score (latent variable) that is highly relevant to compatibility and y is a predicted value of compatibility, is generated, and thus, it is possible to improve the accuracy of a predicted value of compatibility.

Further, since the compatibility score is a score based on a questionnaire result acquired from a plurality of persons in the food industry, it is possible to easily cause the preferences of customers of the respective persons in the food industry to be reflected in the compatibility score.

Further, by narrowing down the target to persons in the food industry in a specific country in its entirety or a specific region in its entirety, it is possible to cause the preferences of persons in the specific country in its entirety or the specific region in its entirety to be reflected in the compatibility score.

For example, compatibility between a predetermined ingredient and a predetermined seasoning may vary in terms of good or bad compatibility depending on persons in a meat-eating country, persons in a vegetarian country, and persons in a country, such as India, where there is no culture of eating beef and the main ingredients are vegetables, beans, fish, or the like, respectively.

The present embodiment makes it possible, by changing the target for the pre-aggregated evaluation of compatibility between an ingredient and a seasoning for each specific country or for each specific region, to perform different compatibility predictions in each specific country or each specific region. As a result, it is possible to provide an appropriate predicted value of compatibility for each specific country or for each specific region.

Next, Embodiment 2 will be described.

In Embodiment 1 described above, one regression equation (compatibility prediction model) is generated by using all index scores and compatibility scores in combinations of all ingredients and all seasonings. In Embodiment 2, ingredients or seasonings may be grouped based on the OAV.

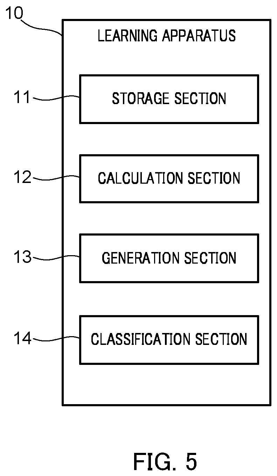

As illustrated in FIG. 5, learning apparatus 10 in Embodiment 2 includes classification section 14 in addition to storage section 11, calculation section 12, and generation section 13.

Classification section 14 groups a plurality of ingredients based on the OAV (first index) for each ingredient, and groups a plurality of seasonings based on the OAV (second index) for each seasoning.

For example, classification section 14 classifies ingredients and seasonings, in which the OAVs for each flavor type are relatively close to each other, into a plurality of groups. For example, a hierarchical cluster analysis technique may be applied as a classification method by classification section 14.

The hierarchical cluster analysis is a technique in which combinations of data that are similar to each other in a data group are sequentially grouped together in descending order of similarity and are ultimately grouped into one cluster. For example, two ingredients or seasonings in which the tendencies of OAV conversion scores for all flavor types are most similar to each other are grouped and all data are grouped to complete one tree diagram. As the grouping technique, for example, a publicly known method, such as a Ward's method, a minimum distance method, a maximum distance method, a centroid method, a group average method, and a median method, may be used.

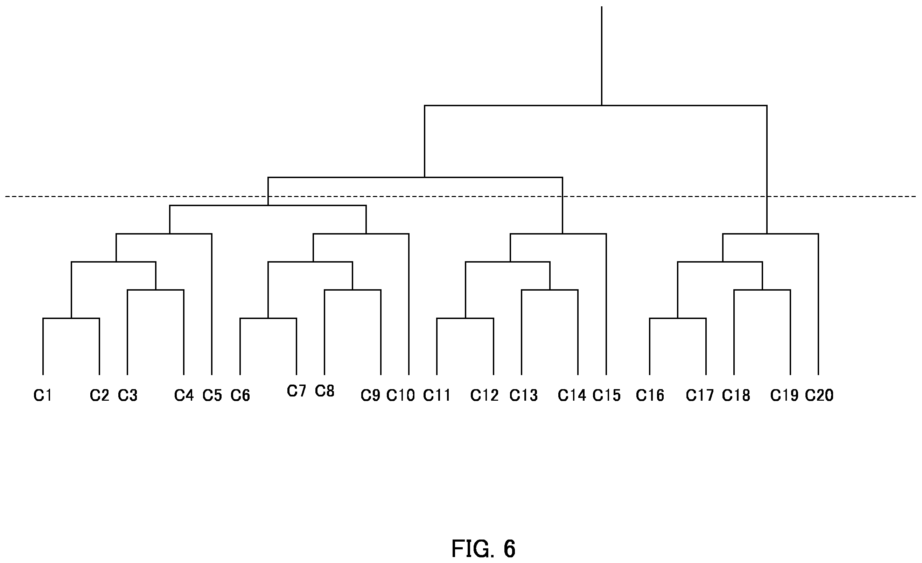

For example, FIG. 6 illustrates a portion of a tree diagram in which a plurality of data is classified by a hierarchical cluster analysis. For convenience, it is assumed that the tree diagram in FIG. 6 is formed of 20 pieces of data ranging from C1 to C20.

For example, in a case where the data are classified into three groups, it is assumed, in a hierarchy (the layer indicated by the broken line) where the tree diagram includes three branches, that data belonging to each branch form one group. For example, in the layer indicated by the broken line in FIG. 6, the group of the leftmost branch includes C1 to C10, the group of the middle branch includes C11 to C15, and the group of the rightmost branch includes C16 to C20.

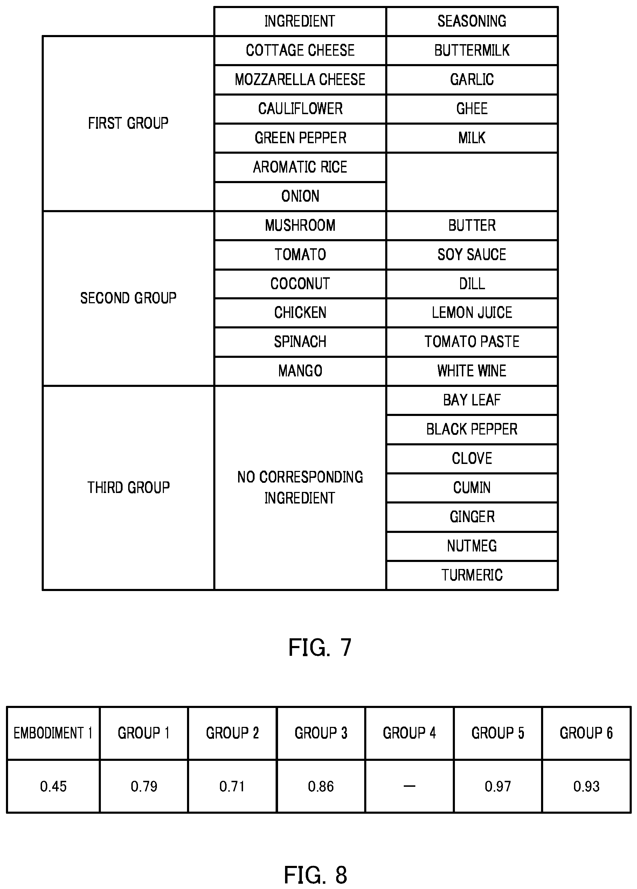

Thus, in a case where the 12 kinds of ingredients described above and the 17 kinds of seasonings described above are classified into three groups as an example, the ingredients and seasonings are classified into three groups of a first group, a second group, and a third group as illustrated in FIG. 7, for example.

The first group is characterized in that, for example, the OAV conversion score for each flavor type is low on the whole, and the aroma is mild and/or monotone on the whole.

The second group is characterized in that, for example, the OAV conversion scores for Fruit and Floral are relatively high and the aroma is complex.

The third group is characterized in that, for example, the OAV conversion scores for Fruit, Floral, Wood, Green, Herb, Spice, and Chemical are very high and an accent is given even in a small amount.

In the case of the 12 kinds of ingredients described above, the cottage cheese, mozzarella cheese, cauliflower, green pepper, aromatic rice, and onion are classified into the first group, and the mushroom, tomato, coconut, chicken, spinach, and mango are classified into the second group. None of the 12 kinds of ingredients is classified into the third group.

Further, in the case of the 17 kinds of seasonings described above, the buttermilk, garlic, ghee, and milk are classified into the first group, the butter, soy sauce, dill, lemon juice, tomato paste, and white wine are classified into the second group, and the bay leaf, black pepper, clove, cumin, ginger, nutmeg, and turmeric are classified into the third group.

Generation section 13 generates a compatibility prediction model for each combination of the groups of ingredients and the groups of seasonings. In other words, a plurality of compatibility prediction models is generated in accordance with combinations of the groups of ingredients and the groups of seasonings.

Specifically, generation section 13 generates a total of six compatibility prediction models: a combination of the first group according to the ingredients and the first group according to the seasonings (group 1); a combination of the first group according to the ingredients and the second group according to the seasonings (group 2); a combination of the first group according to the ingredients and the third group according to the seasonings (group 3); a combination of the second group according to the ingredients and the first group according to the seasonings (group 4); a combination of the second group according to the ingredients and the second group according to the seasonings (group 5); and a combination of the second group according to the ingredients and the third group according to the seasonings (group 6).

Further, FIG. 8 illustrates results of accuracy comparison between predicted values of compatibility in Embodiments 1 and 2, with each of the 12 kinds of ingredients and the 17 kinds of seasonings as arbitrary data.

FIG. 8 illustrates calculation results of values of coefficients of determination with respect to the respective combinations in Embodiments 1 and 2. Note that, the calculation results in FIG. 8 or the like represent results calculated based on data on the aroma components included in the respective ingredients and seasonings acquired from the predetermined databases described above.

In Embodiment 1, the coefficient of determination is 0.45, whereas in Embodiment 2, the coefficients of determination in groups 1, 2, 3, 5, and 6 are 0.79, 0.71, 0.86, 0.97, and 0.93, respectively, each of which has been confirmed to have improved in comparison with that in Embodiment 1.

Note that, no compatibility prediction model has been generated for group 4. This is due to the fact that an ingredient(s) or a seasoning(s) that reduce(s) the prediction accuracy is/are included in a group according to the combinations of the ingredient groups and the seasoning groups. In this case, in Embodiment 2, the predicted value of compatibility for the ingredients or seasonings included in group 4 may be calculated by using the same compatibility prediction model as in Embodiment 1.

In Embodiment 2 configured as described above, ingredients and seasonings, in which the OAVs for each flavor type are relatively close to each other, are grouped, respectively, and a compatibility prediction model is generated for each combination of the groups of the ingredients and the groups of the seasonings, and thus, it is possible to improve the accuracy of compatibility prediction.

Next, Embodiment 3 will be described.

In Embodiment 2 described above, there is a case where no compatibility prediction model is generated when an ingredient(s) or a seasoning(s) that reduce(s) the prediction accuracy is/are included in a group according to the combinations of the ingredient groups and the seasoning groups. In Embodiment 3, in a case where an ingredient(s) or a seasoning(s) that reduce(s) the prediction accuracy is/are included in a group, the ingredient(s) or the seasoning(s) that reduce(s) the prediction accuracy may be excluded from the group.

For example, in a case where an arbitrary one of ingredients and seasonings included in each group is excluded and a compatibility prediction model is generated when the coefficients of determination in the compatibility prediction model improves in comparison with that before the exclusion, the arbitrary one may be specified as an ingredient or a seasoning that reduces the prediction accuracy.

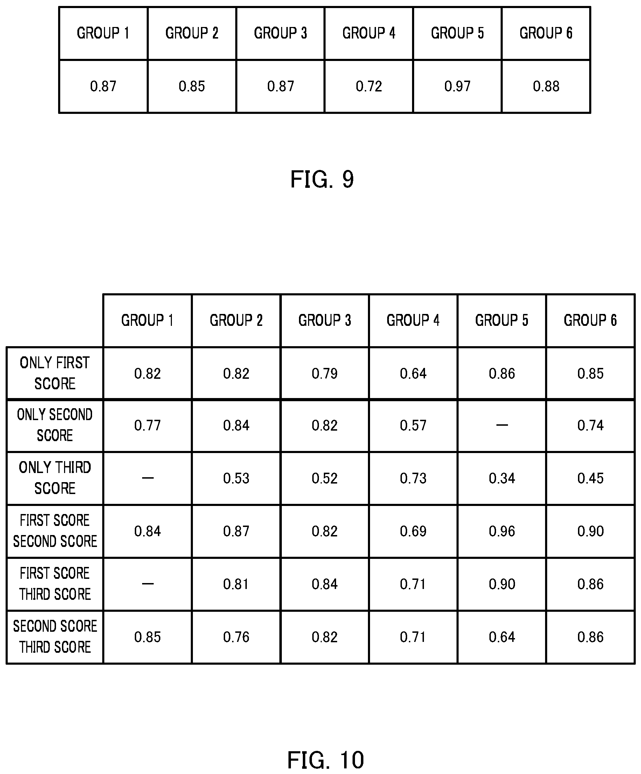

For example, when compatibility prediction models are generated for groups 1 to 3 in Embodiment 2 by excluding an arbitrary one of the ingredients included in the first group according to the ingredients, it is confirmed as illustrated in FIG. 9 that the coefficients of determination improve as 0.87 in group 1, 0.85 in group 2, and 0.87 in group 3 in comparison with those in Embodiment 2 in a case where the Mozzarella cheese is excluded from the first group according to the ingredients.

Further, when a compatibility prediction model is generated for group 4, in which no compatibility prediction model is generated in Embodiment 2, by excluding an arbitrary one in the second group according to the ingredients between the second group according to the ingredients and the first group according to the seasonings, it has been confirmed that a compatibility prediction model can be generated for group 4 in a case where the mango is excluded from the second group according to the ingredients, with the coefficient of determination of 0.72, which is a value better than that in Embodiment 1.

Further, when compatibility prediction models are also generated for groups 5 and 6 by excluding the mango from the second group according to the ingredients, the coefficient of determination for group 5 is 0.97, which is a value unchanged from that in Embodiment 2, and the coefficient of determination for group 6 is 0.88, which is a value lower than that in Embodiment 2. For that reason, in the case of a group in which the prediction accuracy decreases after an ingredient(s) or a seasoning(s) is/are excluded from the group, no ingredient or seasoning may be excluded from the group.

In addition, with respect to an ingredient excluded from a group, a predicted value of compatibility may be calculated by using the same compatibility prediction model or the like as that in Embodiment 2 in a case where the same compatibility prediction model as that in Embodiment 1 is generated. Further, a compatibility prediction model may be generated with a combination of an excluded ingredient and every seasoning.

For example, in a case where a compatibility prediction model is generated for a combination of the Mozzarella cheese and every seasoning, it has been confirmed that the coefficient of determination is 0.97. Further, compatibility prediction models may be generated for combinations of an excluded ingredient and each group of the seasonings.

In Embodiment 3 configured as described above, an ingredient(s) or a seasoning(s) that reduce(s) the prediction accuracy is/are excluded from a group, and thus it is possible to improve the prediction accuracy of a compatibility prediction model for each group.

Note that, although the first score, the second score, and the third score are used as the index score in each embodiment described above, the present invention is not limited thereto. For example, a compatibility prediction model may be generated by using at least one of the first score, the second, and/or the third score.

For example, FIG. 10 illustrates results of calculation of coefficients of determination in a case where compatibility prediction models are generated in each group in Embodiment 2 based on only the first score; only the second score; only the third score; the first score and the second score; the first score and the third score; and the second score and the third score, respectively. Note that, no compatibility prediction model can be generated for only the second score in group 5, as well as for only the third score; and the first score and the third score in group 1.

According to the results illustrated in FIG. 10, the coefficient of determination for the second score and the third score is the largest with 0.85 in group 1, the coefficient of determination for the first score and the second score is the largest with 0.87 in group 2, and the coefficient of determination for the first score and the third score is the largest with 0.84 in group 3.

Further, the coefficient of determination for only the third score is the largest with 0.73 in group 4, the coefficient of determination for the first score and the second score is the largest with 0.96 in group 5, and the coefficient of determination for the first score and the second score is the largest with 0.90 in group 6.

Given these results, it may be configured such that a compatibility prediction model for which the coefficient of determination is the largest is selected and a predicted value of compatibility is calculated.

Further, although a compatibility prediction model is generated based on the 12 kinds of ingredients and the 17 kinds of seasonings in each embodiment described above, the present invention is not limited thereto. The kinds of ingredients may be equal to or greater than 12 or may be less than 12. Further, the kinds of seasonings may be equal to or greater than 17 or may be less than 17.

Further, although the plurality of seasonings is of different kinds in each embodiment described above, the present invention is not limited thereto. The plurality of seasonings may include a plurality of seasonings of the same kind with, for example, different OAVs. For example, the plurality of seasonings may include different kinds of soy sauce (for example, *koikuchi* (dark soy sauce), *usukuchi* (light soy sauce), less sodium, or the like).

Further, although learning apparatus 10 performs learning by using the partial least squares method in each embodiment described above, the present invention is not limited thereto. Learning apparatus 10 may perform learning by using any other method.

Further, although the regression equation generated by using the partial least squares method has been exemplified as the compatibility prediction model in each embodiment described above, the present invention is not limited thereto, and any other model such as a neural network is possible.

Further, in each embodiment described above, prediction apparatus 1 includes storage section 2 that stores a compatibility prediction model, but the present invention is not limited thereto. For example, prediction apparatus 1 may be configured to transmit data, which can be inputted into a compatibility prediction model, to another apparatus (such as a server apparatus) that stores a compatibility prediction model, and receives a predicted value of compatibility.

Further, although the OAV has been exemplified as indexes related to flavors in an ingredient and a seasoning in each embodiment described above, the present invention is not limited thereto. Each index may be a taste activity value (TAV), which is an index indicating the importance of a taste, may be a charm value or an FD factor, which is an index indicating the importance of a flavor, or may be any other index related to a taste or an aroma.

Next, an evaluation experiment performed by using prediction apparatus 1 according to the present embodiment will be described. Specifically, in this experiment, some candidates for a flavor type having a good compatibility with a predetermined ingredient are selected based on a predicted value of compatibility between the predetermined ingredient and a first seasoning by prediction apparatus 1 according to the present embodiment. Then, this experiment has evaluated, when the predetermined ingredient is cooked by using a second seasoning obtained by adding a component of a candidate flavor type to the first seasoning, how the preferences of persons who have eaten the cooked product change.

The predetermined ingredient is, for example, rice used for fried rice, and the first seasoning is soy sauce. The first seasoning is arbitrary soy sauce (for example, soy sauce of company A). First, a predicted value of compatibility between the rice and the soy sauce is calculated by using prediction apparatus 1. The predicted value of compatibility is calculated based on the regression equation as described above. The predicted value of compatibility in a combination of the rice and the soy sauce can be expressed by an equation that indicates the sum of the first score, the second score, and the third score, that is, those obtained by multiplying the 39 scores by predetermined coefficients, respectively.

The coefficients whereby the 39 scores are multiplied, respectively, are values set for the 39 scores, respectively, where the larger the value, the greater the degree of effect on the predicted value of compatibility. For example, a flavor type related to a score with the largest coefficient among the coefficients is selected as a candidate. For example, in a case where the score with the largest coefficient is the second store (the absolute value of a difference value between the respective OAV conversion scores for the rice and the soy sauce) according to Fruit, Fruit becomes a candidate for a flavor type.

Next, soy sauce (second seasoning) to which a component of a flavor type for the score with the largest coefficient described above is added is generated. Further, soy sauce (third seasoning) to which all components of flavor types for scores for which the coefficient described above is equal to or greater than a predetermined value are added is generated. Then, fried rice (first fried rice) cooked with the second seasoning and the rice as well as fried rice (second fried rice) cooked with the third seasoning and the rice are prepared, respectively. Further, fried rice (third fried rice) cooked with the soy sauce (first seasoning) prior to the addition of the component of the flavor type and the rice is prepared as an object for comparison.

Then, questionnaire surveys on two pieces of the cooked fried rice are conducted with the persons in the food industry who have performed the pre-aggregated evaluation of compatibility between an ingredient and a seasoning when a compatibility prediction model in prediction apparatus 1 is generated. The persons in the food industry in this experiment are 53 Indian chefs. Note that, it is assumed that the persons in the food industry who have performed the evaluation of compatibility when the compatibility prediction model in prediction apparatus 1 used in this experiment is generated are a plurality of Indian chefs, and may be the same as or different from the persons in the food industry in this experiment.

The questionnaire surveys that have been conducted are: (1) to compare the first fried rice and the third fried rice to select a preferred fried rice; (2) to compare the second fried rice and the third fried rice to select a preferred fried rice; and (3) to score the degrees of preference for the first fried rice, the second fried rice, and the third fried rice, respectively.

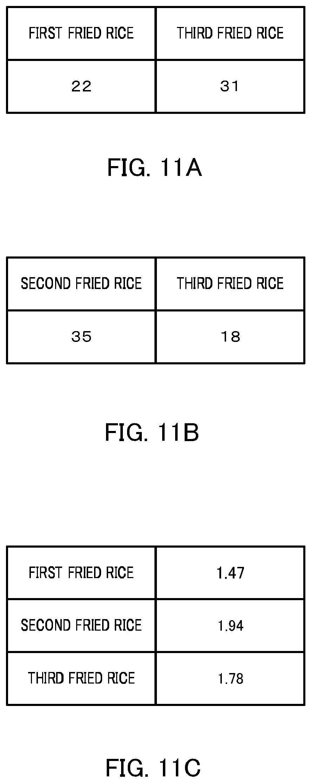

FIG. 11A illustrates results of the questionnaire survey of (1). FIG. 11B illustrates results of the questionnaire survey in (2). FIG. 11C illustrates results of the questionnaire survey in (3).

As illustrated in FIG. 11A, the results in (1) are that, among the 53 Indian chefs, 31 Indian chefs have selected the third fried rice and 22 Indian chefs have selected the first fried rice. Further, as illustrated in FIG. 11B, the results in (2) are that, among the 53 Indian chefs, 18 Indian chefs have selected the third fried rice and 35 Indian chefs have selected the second fried rice.

As seen above, it has been confirmed that the preference varies when a component of a flavor type deemed to have a good compatibility with a predetermined ingredient is added to a seasoning and cooking is performed.

Further, in (3), the second fried rice (the fried rice cooked with the third seasoning, to which components of a plurality of flavor types are added, and the rice) has obtained the best score among the first fried rice, the second fried rice, and the third fried rice as illustrated in FIG. 11C. The scores illustrated in FIG. 11C are the average values of scores for the respective pieces of fried rice evaluated by each of the 53 Indian chefs with nine levels from -4 to 4. The value of the score indicates that the larger the value, the higher the preference.

Further, it has been confirmed that each fried rice has a positive value, which allows it to be confirmed that the preference is relatively high in each fried rice.

Next, each of FIGS. 12A and 12B illustrates a breakdown of the respective numbers of Indian chefs who select one of the pieces of fried rice in (2), where the Indian chefs have selected the first fried rice or the third fried rice in (1).

As illustrated in FIG. 12A, the results are that, among the 31 Indian chefs who have selected the third fried rice in (1), 17 Indian chefs have selected the second fried rice in (2), with the number thereof being larger than the number of Indian chefs who have selected the third fried rice in (2). Further, as illustrated in FIG. 12B, the results are that, among the 22 Indian chefs who have selected the first fried rice in (1), 18 Indian chefs have selected the second fried rice in (2), with the number thereof being significantly larger than the number of Indian chefs who have selected the third fried rice in (2).

Given the above, it is possible to confirm that the preference improves by adding components of a plurality of kinds of flavor types to a seasoning.

Further, FIG. 13 illustrates aggregate results of scores of pieces of fried rice selected in each group, which are obtained by grouping Indian chefs who have indicated the same preference in FIGS. 12A and 12B.

The A group illustrated in FIG. 13 is a group of Indian chefs who have selected the third fried rice in both (1) and (2), which is a group of 14 Indian chefs. The B group illustrated in FIG. 13 is a group of Indian chefs who have selected the first fried rice in (1) and the second fried rice in (2), which is a group of 18 Indian chefs. The C group illustrated in FIG. 13 is a group of Indian chefs who have selected the third fried rice in (1) and the second fried rice in (2), which is a group of 17 Indian chefs. The D group illustrated in FIG. 13 is a group of Indian chefs who have selected the first fried rice in (1) and the third fried rice in (2), which is a group of four Indian chefs.

The results illustrated in FIG. 13 allow it to be confirmed that the scores of the pieces of fried rice selected in each group substantially exceed two. Accordingly, the use of a seasoning obtained by adding a component of a flavor type having a good compatibility to a seasoning makes it possible to realize a cooked product with an improved preference for a person who does not like a cooked product with the original seasoning (the first seasoning) very much. That is, the use of prediction apparatus 1 in the present embodiment makes it easier to extract a flavor type that is added to a seasoning, and further to produce a new seasoning that match the preferences of persons in a specific country or a specific region.

**In** addition, any of the embodiments described above is only illustration of an exemplary embodiment for implementing the present disclosure, and the technical scope of the present disclosure shall not be construed limitedly thereby. That is, the present disclosure can be implemented in various forms without departing from the gist or the main features thereof.

The disclosure of Japanese Patent Application No. 2022-152725, filed on September 26, 2022, including the specification, drawings and abstract, is incorporated herein by reference in its entirety.

### Industrial Applicability

The prediction apparatus of the present disclosure is useful as a predictable prediction apparatus, a learning apparatus, and a learning method each capable of predicting compatibility between an ingredient and a seasoning in consideration of the preferences of persons who have a meal.

### Reference Signs List

1 Prediction apparatus
2 Storage section
3 Input section
4 Processing section
5 Output section
10 Learning apparatus
11 Storage section
12 Calculation section
13 Generation section

## Claims

1. A prediction apparatus, comprising:
an input section that allows information on an ingredient and a seasoning to be inputted into the input section;
a processing section that extracts an index score based on the information inputted into the input section, the index score being inputted into a compatibility prediction model for the ingredient and the seasoning, the compatibility prediction model having been learned based on the index score and a compatibility score, wherein
the index score is based on a first index and a second index, wherein
the first index is calculated based on data on a flavor in the ingredient, and
the second index is calculated based on data on a flavor in the seasoning, and
the compatibility score indicates compatibility between the ingredient and the seasoning based on a pre-aggregated evaluation result of the compatibility between the ingredient and the seasoning; and
an output section that outputs the compatibility score as a predicted value of the compatibility between the ingredient and the seasoning, the compatibility score having been outputted from the compatibility prediction model by inputting the index score into the compatibility prediction model, the index score having been extracted by the processing section.

2. The prediction apparatus according to claim 1, wherein a plurality of the index scores is calculated in accordance with a plurality of flavor types, respectively.

3. The prediction apparatus according to claim 1, wherein the index score includes at least one of a first score, a second score, and/or a third score, the first score being a value obtained by adding the first index and the second index, the second score being an absolute value of a difference between the first index and the second index, the third score being related to an off-flavor in the second index.

4. The prediction apparatus according to claim 1, wherein:
a plurality of the first indexes is calculated in accordance with a plurality of the ingredients, respectively,
a plurality of the second indexes is calculated in accordance with a plurality of the seasonings, respectively, and
a plurality of the index scores is calculated in accordance with a combination of the first index of each of the plurality of ingredients and the second index of each of the plurality of seasonings.

5. The prediction apparatus according to claim 4, wherein:
the plurality of ingredients is grouped based on the plurality of first indexes, respectively,
the plurality of seasonings is grouped based on the plurality of second indexes, respectively, and
a plurality of the compatibility prediction models is generated in accordance with combinations of groups of the plurality of ingredients and groups of the plurality of seasonings.

6. A learning apparatus, comprising:
a calculation section that calculates an index score based on a first index and a second index, the first index being calculated based on data on a flavor in an ingredient, the second index being calculated based on data on a flavor in a seasoning; and
a generation section that performs learning based on the index score and a compatibility score, and generates a compatibility prediction model for the ingredient and the seasoning, the compatibility score indicating compatibility between the ingredient and the seasoning based on a pre-aggregated evaluation result of the compatibility between the ingredient and the seasoning.

7. A learning method, comprising:
calculating an index score based on a first index and a second index, the first index being calculated based on data on a flavor in an ingredient, the second index being calculated based on data on a flavor in a seasoning; and
performing learning based on the index score and a compatibility score, and generating a compatibility prediction model for the ingredient and the seasoning, the compatibility score indicating compatibility between the ingredient and the seasoning based on a pre-aggregated evaluation result of the compatibility between the ingredient and the seasoning.
